# EUROPEAN PATENT SPECIFICATION

(11) **EP 0 274 277 B1**
(45) Date of publication and mention of the grant of the patent: **27.01.1993**
(21) Application number: 87311519.0
(22) Date of filing: 30.12.1987
(51) Int. Cl.: C12P 17/14, C12P 41/00

(54) **A process for the enzymatic separation of the optical isomers of racemic oxazolidinonic derivatives**
Verfahren zur enzymatischen Trennung der optischen Isomere von racemischen Oxazolidinon-Derivaten
Procédé de séparation enzymatique d'isomères optiques de dérivés racémiques d'oxazolidinone

(30) Priority: 30.12.1986 IT 2288486
(43) Date of publication of application: 13.07.1988
(73) Proprietor: Montedison S.p.A., I-20121 Milan (IT)
(72) Inventor: Cesti, Pietro, I-28069 Trecate Novara (IT); Bianchi, Daniele, I-20149 Milan (IT); Francalanci, Franco, I-28100 Novara (IT); Cabri, Walter, I-20051 Limbiate, Milan (IT)
(74) Representative: Lamb, John Baxter

(56) References cited:
- EP-A- 0 101 076

## Description

The present invention relates to a process for the separation, using enzymes, of the S(+) and R(-) optical isomers of racemic derivatives of oxazolidinone.

More particularly, the invention relates to a process for the separation or resolution of the optical isomers of 3-alkyl-5-hydroxymethyl-oxazolidin-2-ones of the formula: 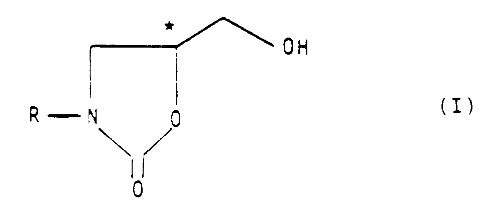
(in which R is a straight or branched C₁-C₈ alkyl group). Compounds of formula (I) are usually in the form of a mixture of the S(+) and R(-) optical isomers. The process of the invention is carried out using enzymes having esterasic activity and derived from micro-organisms or from animal tissues.

Compounds of formula (I), in the form of the S(+) or R(-) optical isomers or of their racemic mixtures, are important intermediates for use in the synthesis of β-blocking drugs (Agric. Biol. Chem., 49 (1). 207-210, 1985), for instance according to a process as represented schematically below for the S(+) optical isomer:

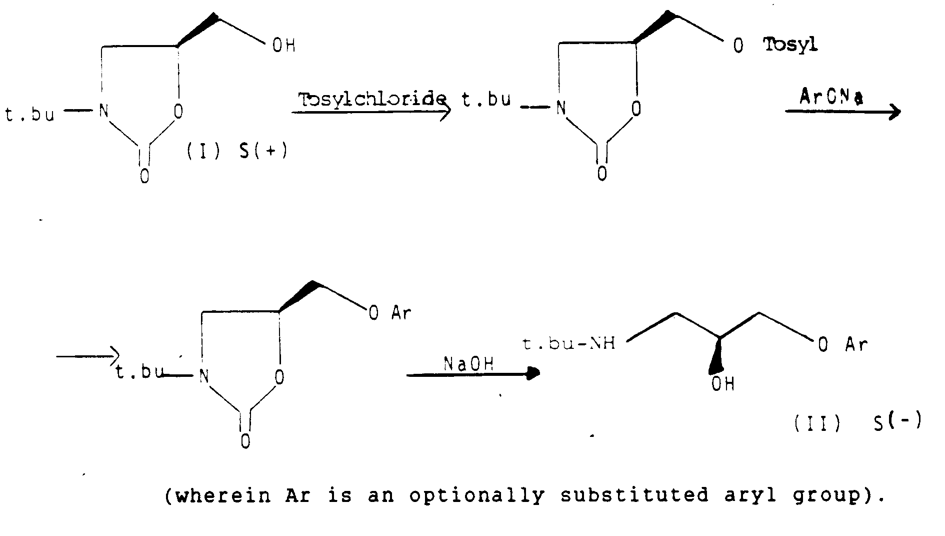

Thus, the compounds of formula (II) are obtained in their optically active S(-) form, which compounds are β-blocking agents. The compounds of formula (II) are, however, used in clinical practice in the form of racemes, though the S(-) isomer has higher activity than the R(=) isomer. (Nature 210, 1336, 1966).

Clearly, therefore, it is desirable to have an effective process for the separation of the optically active forms of racemic compounds of formula (I).

Some processes are known for the synthetic selective Preparation of compounds of formula (I) in optically active form.

Thus, there has been proposed (Chem. Pharm. Bull. 29,3593-3600, 1981; J. Org. Chem. 43, 3641, 1978) a process basically comprising the steps of oxidizing D-mannitol in the presence of Pb tetraacetate to give D-glyceraldehyde acetonide; treating the acetonide with a primary amine to give a 3-alkylamino-1,2-propanediol; and finally, cyclizing the diol to give a product (I) in the optically active S(+) form. This method is not particularly suitable for industrial use owing to the various tedious operating steps involved and owing to the use of considerable amounts of lead tetraacetate in the first step to produce D-glyceraldehyde acetonide from D-mannitol.

There has also been described (Agr. Biol Chem. 48, 2055-2059, 1984; EP-A- 0101076) a process starting from a compound of formula (I) in racemic form. The compound (I) is chemically acetylated and then the esterified R(-) form, thus obtained, is selectively enzymatically hydrolysed. The esterified S(+) form of the compound of formula (1) is then separated and subsequently chemically hydrolyzed to give the S(+) form of the compound (I).

However, this method also entails several operating steps and is not particularly attractive from an industrial point of view. In fact, the process involves two hydrolysis operations, one enzymatic and one chemical, besides the initial acetylation step.

It is an object of the present invention to provide a process for carrying out the separation or resolution of optical isomers of racemic oxazolidinone derivatives of formula (I) to directly give the S(+) isomer, which is of particular interest.

It has now been found, in accordance with the present invention, that this object can be attained by the use of an enzymatic asymmetric esterification of racemic compounds of formula (I), using particular enzymes as hereinafter described more particularly.

In practice use is made of enzymes of the lipase class, which enzymes can give rise to a stereo-selective esterification of the R(-) form only of compounds of formula (I), while leaving the isomer in the S(+) form unchanged, which latter is then readily separated for direct use.

According to the invention there is provided a process for the separation or resolution of racemic mixtures of the S(+) and R(-) optical isomers of oxalidinone compounds of the formula: 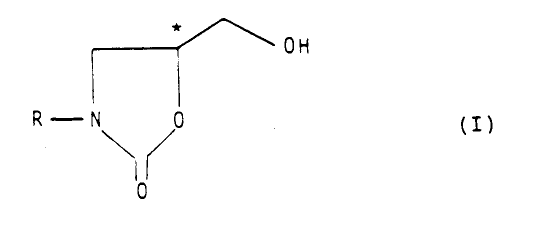
which process comprises reacting a racemic 3-alkyl-5-hydroxymethyl-oxazolidin-2-one of formula (I) with an esterifying compound which is
(a) an ester of the formula: 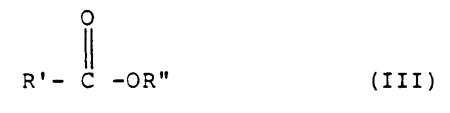 [in which R¹ is a straight or branched C₁-C₁₀ alkyl or alkenyl group and Rʺ is a straight or branched C₁-C₄ alkyl or alkenyl group, a haloalkyl (e.g. chloroalkyl or bromoalkyl) or a diacylglycerolic group].
(b) an acid of the formula:

   Rʺʹ - COOH (IV)

   (in which Rʺʹ is a straight or branched E₁-C₂₀ alkyl or alkenyl group); or
   an anhydride of the formula: 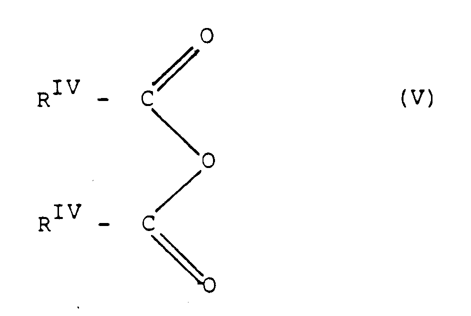 (in which R^{IV} is a straight or branched C₁-C₆ alkyl group);
   the reaction being carried out in the presence of an enzyme, immobilized on a porous carrier, capable of selectively giving rise to the esterification reaction of the R(-) isomer, while leaving the S(+) isomer substantially unchanged; and
   separating the unchanged S(+) isomer.

The racemic 3-alkyl-5-hydroxymethyl-ozazolidin-2-ones of formula (I), used as starting material, are known compounds and/or may be synthethized by conventional techniques.

As shown schematically below, in the process of the invention, the racemic oxazolidinonic compound of formula (I), is reacted, in the presence of an enzyme, preferably of the lipase class, in an organic solution, with an ester of formula (III), an acid of formula (IV) or an anhydride of formula (V) (as defined above).

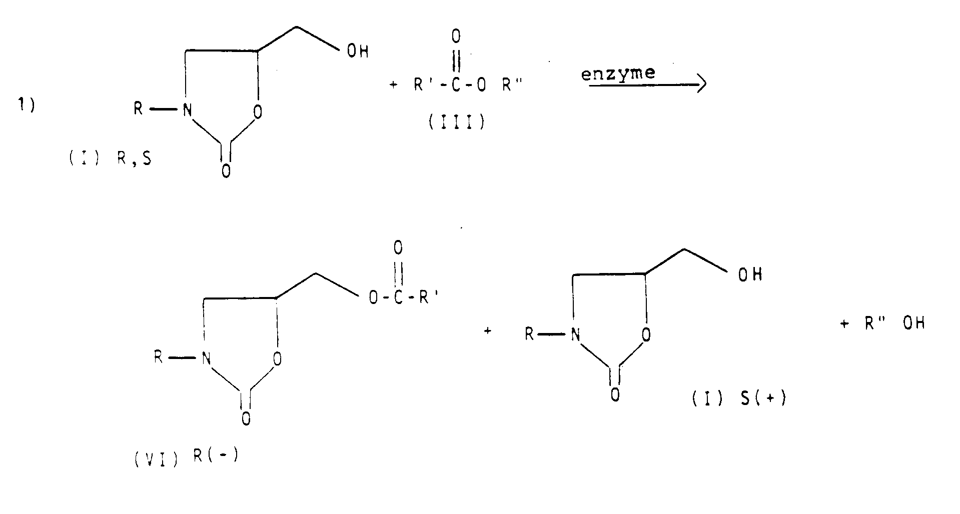

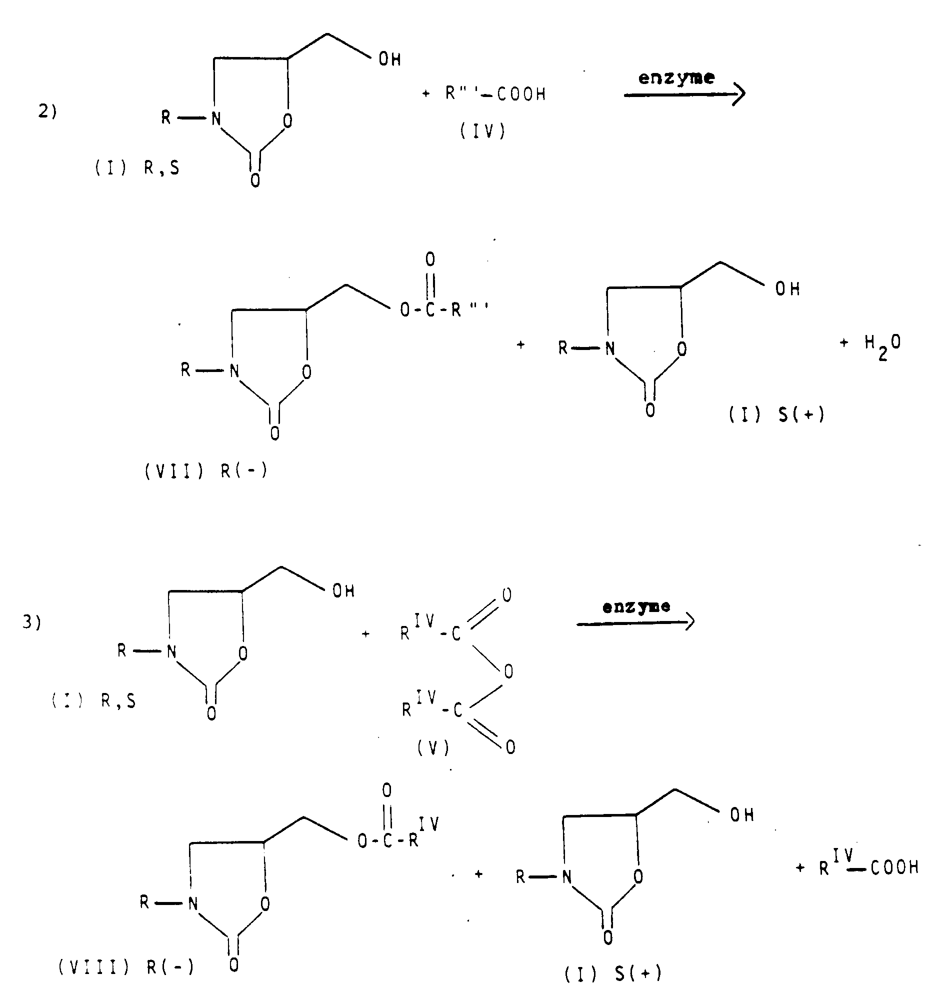

The process shown in reaction (1) is an enzymatic transesterification reaction, in which the starting racemic compound of formula (I) is reacted with an ester of formula (III), in the presence of an enzyme. Preferred esters of formula (III) are ethyl acetate, trichloroethyl butyrate and glycerol tributyrate (tributyrin).

The reaction is suitably carried out using a stoichiometric excess of ester (III) which can then serve, not only as a reagent, but also as a solvent medium. In particular, the molar ratio of ester (III) to oxazolidinonic compound (I) is suitably from 10:1 to 500:1, preferably from 50:1 to 200:1. The enzyme is suitably employed in an amount such that the weight ratio of enzyme to compound of formula (I) is from 1:1 to 1:2000. The molar concentration (M) of the oxazolidinone of formula (I) in the reaction mixture is suitably from 0.01M to 2M, preferably from 0.1M to 1M.

The transesterification reaction is suitably carried out by strongly stirring a reaction mixture consisting of reagent (I), ester (III), excess ester (III) (serving as solvent) and the supported enzyme, as specified hereinafter, at a temperature of from 0° to 50°C, preferably about 20° to 30°C. The reaction time can range from 1 to 72 hours, according to the selected operating conditions.

At the end of the reaction there is filtered off the solid phase consisting essentially of the immobilized enzyme, which can be recovered substantially without loss of activity.

The S(+) 3-alkyl-5-hydroxymethyl-oxazolidin-2-one derivative is separated from the filtrate and the R(-) 3-alkyl-5-acyl-oxymethyl-oxazolidin-2-one derivative by conventional means such as column chromatography, fractional distillation or, more advantageously, by exploiting solubility differences in water and the like.

Reaction (2) is an enzymatic esterification, in which the starting racemic compound of formula (I) is reacted with a carboxylic acid of formula (IV), in the presence of an enzyme. Preferred acids of formula (IV) are octanoic acid, decanoic acid and lauric acid.

The reaction is carried out in an organic solvent which is preferably an aromatic hydrocarbon (e.g. benzene, toluene or the like) or a halogenated aliphatic hydrocarbon (e.g. methylene chloride, chloroform or the like).

The molar ratio of acid (IV) to oxazolidinonic compound (I) is suitably from 0.6:1 to 5:1, preferably from 0.8:1 to 1.5:1. The enzyme is suitably employed in an amount such that the weight ratio of enzyme to compound of formula (I) is from 1:1 to 1:2000. The molar concentration (M) of the oxazolidinone of formula (I) in the reaction mixture is suitably from 0.01M to 2M, preferably from 0.1M to 1M, according to the compound (I) used.

The reaction itself, and separation of the desired product, are suitably carried out as described above in connection with reaction (1).

Reaction (3) is an enzymatic esterification reaction using an anhydride, in which reaction the starting racemic compound of formula (I), is reacted with an anhydride of formula (V), in the presence of an enzyme. Preferred anhydrides are acetic anhydride and propionic anhydride.

The reaction is carried in an organic solvent, preferably an aromatic hydrocarbon or halogenated aliphatic hydrocarbon as discussed above in connection with reaction (2). Similarly, as in reaction (2), the molar ratio of anhydride (V) to oxazolidinone (I) is suitably from 0.6:1 to 5:1, preferably from 0.8:1 to 1.5:1, and the weight ratio of enzyme to compound of formula (I) is suitably from 1:1 to 1:2000. The molar concentration of the oxazolidinone of formula (I) in the reaction mixture is suitably from 0.01M to 2M, preferably from 0.1 to 2M, according to the compound (I).

The esterification reaction is carried out by strongly stirring the reaction mixture comprising reagent (I), reagent (V), the solvent and the supported enzyme, at a temperature of from -10° to 30°C, preferably from 0° to 20°C. The reaction time can range from about 10 minutes to 24 hours, according to the selected operating conditions.

At the end of the reaction, after having removed excess anhydride (V) by means of an aqueous solution of an alkaline carbonate, the reaction mixture is worked up as described above in connection with reaction (1).

In order to carry out the process of the present invention, use can be made of enzymes having hydrolytic action, which are commercially available, of different origins (e.g. derived from microorganisms or animal tissues). Preferably belonging to the lipase class.

Among these enzymes, the following have proved to be particularly active:

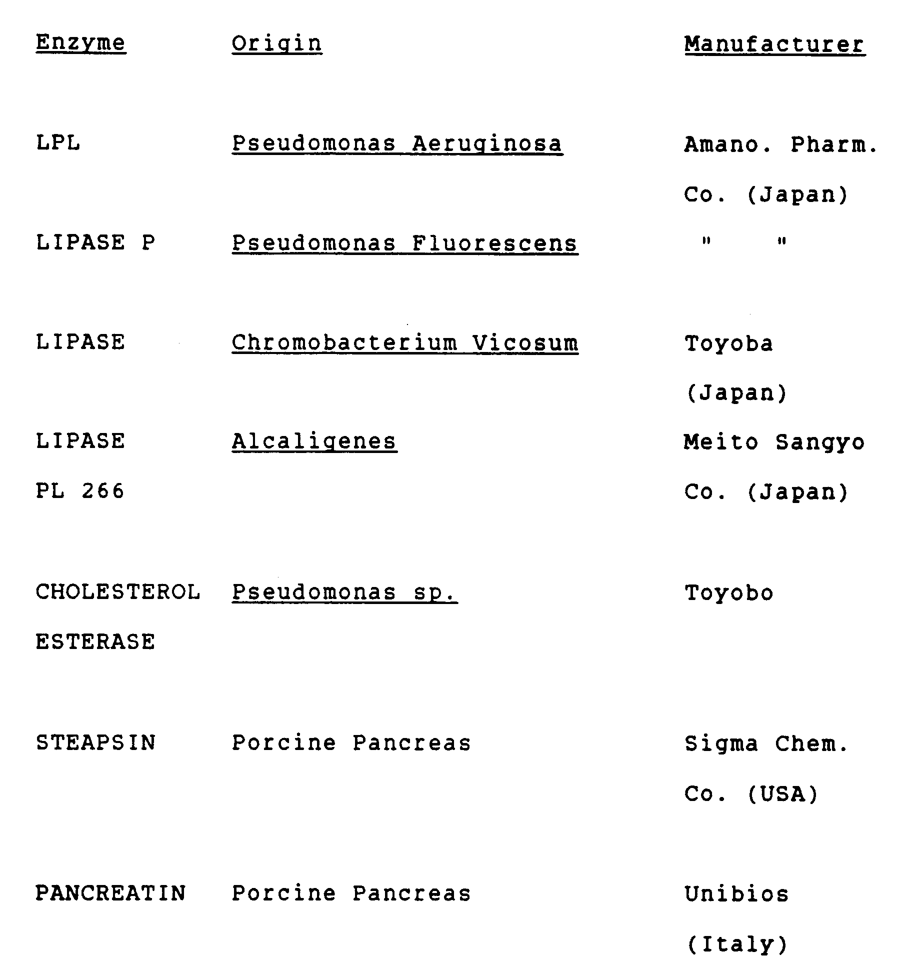

Of the above, the following enzymes are most preferred: LPL, LIPASE P, LIPASE from Chromobacterium Viscosum, LIPASE PL 266.

The enzymes for use in accordance with the invention are immobilized on suitable carriers or substrates in order to increase their stability and to facilitate their recovery and further use.

Porous carriers having a high surface area have proved to be particularly suitable for this purpose and examples of such carriers are diatomaceous earths, alumina, silica, acrylic resins, polystyrene resins and phenol-formaldehyde resins. The immobilization can be easily effected by allowing an aqueous buffer solution containing the enzyme to be absorbed by the porous carrier and then drying the carrier.

The process of the invention, on account of its simple and gentle operating conditions, proves to be particularly advantageous. A particular aspect of high interest consists in the possibility of operating according to an one-step process, leading to the direct separation of the desired S(+) form from the R(-) form, with high yields and with good purity.

In order that the invention may be well understood the following Examples are given by way of illustration only.

### EXAMPLE 1

### Enzyme immobilization

25 mg of enzyme L.P.L. Amano 100S (lipoprotein lipase EC 3.1.1.4, from Pseudomonas aeruginosa; Amano Pharmaceutical Co. Ltd; 1.120 units per mg) dissolved in 3 ml of a buffer solution (Na/K phosphate 0.1N at pH = 7), were added to 500 mg of Celite 577® (Johns - Manville Ltd. Richmond, Surrey).

The mixture was stirred, in order to obtain a uniform distribution of the enzyme, and was then dried in air at 20°C over 24 hours.

### Transesterification reaction

### Separation of the R(-) and S(+) isomers of 3-tert.butyl-5-hydroxymethyl oxazolidin-2-one.

5 g of (R)(S)-3-tert.butyl-5-hydroxymethyl oxazolidin-2-one and 500 mg of Celite® containing the immobilized enzyme, were added to 200 ml of ethyl acetate. The mixture was stirred vigorously at 20 °C and the progress of the reaction was checked by gas chromatography. After 6 hours (50% conversion) the enzyme was recovered by filtration and ethyl acetate was evaporated off at reduced pressure.

The residue was then analysed by chromatography on a silica gel column, by eluting with a 7:3 ethyl acetate-hexane mixture.

There were obtained 2.8 g of (R)-(-)-3-tert.butyl-5-acetoxymethyloxazolicin-2-one, as a colourless oil with [α¹⁶ _{D}-36.2°-(C=1.0, CHCl₃), ¹H-NMR (90MH_{Z} in CDCl₃) δ(ppm): 1.4 (9H, s, (CH₃)_{3C}-), 2.2(3H, s, CH₃CO-), 3.35∼3.85 (2H, m, -CH₂N-), 4.1∼4.25(2H, m, CH₂O-), 4.45∼4.75(1H, m, CH₂CH(O-)CH₂); and 2.3 g of S-(+)-3-tert.butyl-5-hydroxylmethyloxazolidin-2-one, as a white solid with [α]¹⁶ _{D}+ 46.0° (C=1, CHCl₃), (after crystallisation from ethyl acetate-hexane 1:1), ¹H-NMR(90MH_{Z} in CDCl₃) δ(ppm): 1.4 (9H, s, (CH₃)₃C-), 3.4∼3.95 (5H, m, -CH₂N-, -CH₂O-, -OH), 4.3∼4.6(1H, m, -CH₂CH (O-)CH₂).

The (R)-(-)-3-tert.butyl-5-acetoxymethyloxazolidin-2-one was hydrolyzed at pH 12 with aqueous sodium hydroxide. On completion of the hydrolysis the mixture was extracted with 50 ml of ethyl acetate, the organic phase was dehydrated and the solvent was evaporated off under reduced pressure. 2.2 g of R-(-)-3-tert,butyl-5-hydroxymethyloxazolidin-2-one were thus obtained, as a white solid with [δ]¹⁶ _{D} -45.9° after crystallization.

### Enzyme recycle

The recovered enzyme was used again for two further consecutive reaction cycles under the same conditions without any appreciable activity loss.

### EXAMPLES 2-11

The procedure of Example 1 was repeated, while changing the ester used for the transesterification, the enzyme and the inert carrier.

The results are set forth in Table 1 below.

### EXAMPLE 12

### Transesterification reaction

### Separation of the R(-) and S(+) enantiomers of 3-isopropyl-5-hydroxymethyloxazolidin-2-one.

5 gram of 3-isopropyl-5-hydrozymethyloxazolidin-2-one and 1 g of Celite 577® containing 250 mg of enzyme Lipase P (from Pseudomonas Fluorescens, Amano Pharmaceutical Co., Ltd,. 30 units per mg), immobilized as described in Example 1, were added to 200 ml of 2,2,2-trichloroethylbutyrate.

The mixture was vigorously stirred at 20°C over 6 hours (50% conversion) and it was then processed as described in Example 1.

There were obtained 3.4 g of R(-)-3-isopropyl-5-butyryloxymethyloxazolidin-2-one, as a colourless oil, ¹H-NMR (90MH_{Z} in CDCl₃) δ)ppm): 0.75∼2.5 (13H, m, C₃H₇-, (CH₃)₂CH-), 3.2∼4.85(6H, m, -CH₂N-, CH₂O-, (CH₃)₂CH-, -CH₂CH(O-)CH₂-); and 2.4 g of S-(+)-3-isopropyl-5-hydroxymethyl-oxazolidin-2-one, as a white solid with [α ²⁰ _{D} + 55.3°(C = 1, in CHCl₃). after crystallization from hexane-ethyl acetate 1:1, ¹M-NHR, (90 MH_{Z} in CDCl₃) δ(ppm): 1.2 (6H, d, -CH(CH₃)₂), 3.4∼4.2 (6H, m, -CH₂N-, (CH₃)₂CH, -OH), 4.3∼4.7(1H, m, -CH₂CH(O-)CH₂-).

By hydrolyzing the (R)-(-)-3-isopropyl-5-hydroxymethyloxazolidin-2-one as a white solid with [α]²⁰ _{D} -55.3° (C = 1, in CHCl₃) after crystallization.

### EXAMPLES 13-18

The procedure of Example 12 was repeated, while changing the ester used for the transesterification, the enzyme and the inert carrier.

The results are set forth in Table II below.

### EXAMPLE 19

### Esterification reaction

### Separation of the R(-) and S(+) enantiomers of 3-tert.butyl-5-hydrozymethyloxazolidin-2-one.

5 g of 3-tert.butyl-5-hydroxymethyloxazolidin-2-one, 4.1 g of n-octanoic acid and 25 mg of enzyme LPL immobilized on 500 mg of Celite 577®, (as described in Example 1) were added to 100 ml of benzene.

The mixture was vigorously stirred at 20 °C and the progress of the reaction was checked by gas chromatography.

After 24 hours (48% conversion) the enzyme was recovered by filtration and benzene was evaporated off at reduced pressure. The residue was analysed by chromatography on a silica gel column, by eluting with a 7:3 ethyl acetate-hexane mixture.

There were obtained 4 g of R-(-)-3-tert.butyl-5-octanoyloxymethyloxazolidin-2-one, as a colourless oil, ¹H-NHR(90MH₂ in CDCl₃) δ(ppm): 0.7∼0.25 (24H, m, C₇H₁₅, (CH₃)₃C), 3.3∼3.85 (2H, m, -CH₂N-), 4.15∼4.3(2H, m, -CH₂O-), 4.45∼4.75 (1H, m, CH₂CH(0)CH₂-); and 2.4 g di S-(+)-3-tert.butyl-5-hydroxymethyloxazolidin-2-one, as a white solid with [α]²⁰ _{D} + 45.7° (C = 1 in CHCl₃) after crystallization from hexane-ethyl acetate, 1:1.

The R-(-)-3-tert.butyl-5-octanoyloxymethyl-oxazolidin-2-one was hydrolyzed with aqueous sodium hydrochloride to give 2.1 g. of R-(-)-3-tert.butyl-5-hydroxymethyloxazolidin-2-one, as a white solid, with [α]²⁰ _{D} -45.1° (C = 1 in CHCl₃) after crystallization.

### EXAMPLES 20-25

The procedure of Example 19 was repeated, while changing the acid used for the esterification, the enzyme and the inert carrier.

The results are set forth in Table III below.

### EXAMPLE 26

### Esterification reaction

### Separation of the R(-) and S(+) enantiomers of 3-isopropyl-5-hydroxymethyloxazolidin-2-one

5 g of 3-isopropyl-5-hydroxymethyloxazolidin-2-one, 4 g of octanoic acid and 250 mg of lipase P, immobilized on 1 g of Celite 577® as described in Example 1, were added to 100 ml of benzene. The mixture was vigorously stirred at 20°C and the progress of the reaction was checked by chromatography. After 24 hours (about 50% conversion) the enzyme was recovered by filtration and benzene was evaporated off at reduced pressure. The residue was analysed by chromatography on a silica gel column, eluting with a 7:3 ethyl acetate-hexane mixture.

There were obtained 4.0 g of R(-)-3-isopropyl-5-octanoyloxymethyloxazolidin-2-one, as a colourless oil, ¹H-NMR (90M-M₂ in CDCl₃) δ(ppm) : 0.75∼2.5 (21H, m, c₇H₁₃, (CH₃)₂CH-), 3.2∼3.75(2H, m, -CH₂N-), 3.95∼4.4(3H, m, -CH₂O-, (CH₃)₂CH-), 4.55∼4.85(1H, m, -CH₂CH(O-)CH₂-); and 2.3 g of S-(+)-3-isopropyl-5-hydroxymethyloxazolidin-2-one, as a white solid with [α]²⁰ _{D} +55.4° (C = 1 in CHCl₃) after crystallization from hexane ethyl acetate, 1:1.

The R-(-)-3-isopropyl-5-octanoyloxymethyloxazolidin-2-one was hydrolyzed with aqueous sodium hydroxide to give 2 g of R-(-)-3-isopropyl-5-hydroxymethyloxazolidin-2-one, as a white solid with [α]²⁰ _{D} -55.3° after crystallization.

### EXAMPLES 27-30

The procedure of Example 26 was repeated, while changing the acid used for the esterification, the enzyme and the inert carrier.

The results are set forth in Table IV below.

### EXAMPLE 31

### Esterification reaction by means of anhydrides

### Separation of the R(-) and S(+) enantiomers of 3-tertbutyl-5-hydrozymethyloxazolidin-2-one.

5 g of 3-tert.butyl-5-hydroxylmethyloxazolidin-2-one, 25 mg of enzyme LPL immobilized on 500 mg of Celite 577®, according to the procedure of Example 1, and 2 g of acetic anhydride were added 100 ml of methylene chloride. The mixture was stirred vigorously and the reaction was monitored by chromatography. After 3 hours (50% conversion) the enzyme was recovered by filtration. The solution was then washed with a saturated sodium carbonate solution. The methylene chloride was then dehydrated over sodium sulphate and evaporated off at reduced pressure.

The residue was analysed by chromatography on a silica gel column, eluting with a 7:3 ethyl acetate-hexane mixture.

There were obtained 2.8 g of R(-)-3-tert.butyl-5-acetoxymethyloxazolidin-2-one, as a colourless oil and 2.4 g of S(+)-3-tert.butyl-5-hydroxymethyloxazolidin-2-one, as a white solid with [α]²⁰ _{D} (C = 1, in CHCl₃) + 45.9° after crystallization from ethyl acetate-hexane 1:1.

The R-(-)-3-tert.butyl-5-acetoxymethyloxazolidin-2-one was hydrolyzed with aqueous sodium hydroxide to give 2 g of R-(-)-3-tert.butyl-5-hydroxymethyl-oxazolidin-2-one, as a white solid with [α]²⁰ _{D} (C = 1, CHCl₃) -46.0° after crystallization.

### EXAMPLE 32

### Esterification reaction by means of anhydrides

### Separation of the R(-) and S(+) enantiomers of 3-isopropyl-5-hydroxymethyloxazolidin-2-one.

5 g of 3-isopropyl-5-hydroxymethyloxazolidin-2-one, 250 mg of Lipase P immobilized on 1 g of Celite 577®, as described in example 1, and 2.6 g of acetic anhydride were added to 100 ml of benzene. The mixture was stirred vigorously and the reaction was monitored by chromatography. After 3 hours (48% conversion) the enzyme was recovered by filtration. The benzene solution was washed with saturated sodium carbonate solution, dehydrated over sodium sulphate and the solvent was evaporated off at reduced pressure.

The residue was analysed by chromatography on a silica gel column, eluting with a 7:3 ethyl acetate-hexane mixture.

There were obtained 2.7 g of R-(-)-3-isopropyl-5-acetoxymethyloxazolidin-2-one, as a colourless liquid and 2.2.g of S(+)-3-isopropyl-5-hydroxymethyloxazolidin-2-one, as a white solid, with [α]²⁰ _{D} (C = 1 in CHCl₃) +55.4° after crystallixation from hexane/ethylacetate 1:1.

The R(-)-3-isopropyl-5-acetoxymethyloxazolidin-2-one was hydrolised with aqueous sodium hydroxide to give 2 g of R-(-)-3-isopropyl-5-hydroxymethylosaxolidin-2-one, as a white solid with [α]²⁰ _{D} (C = 1, in CHCl₃) -54.2° after crystallization.

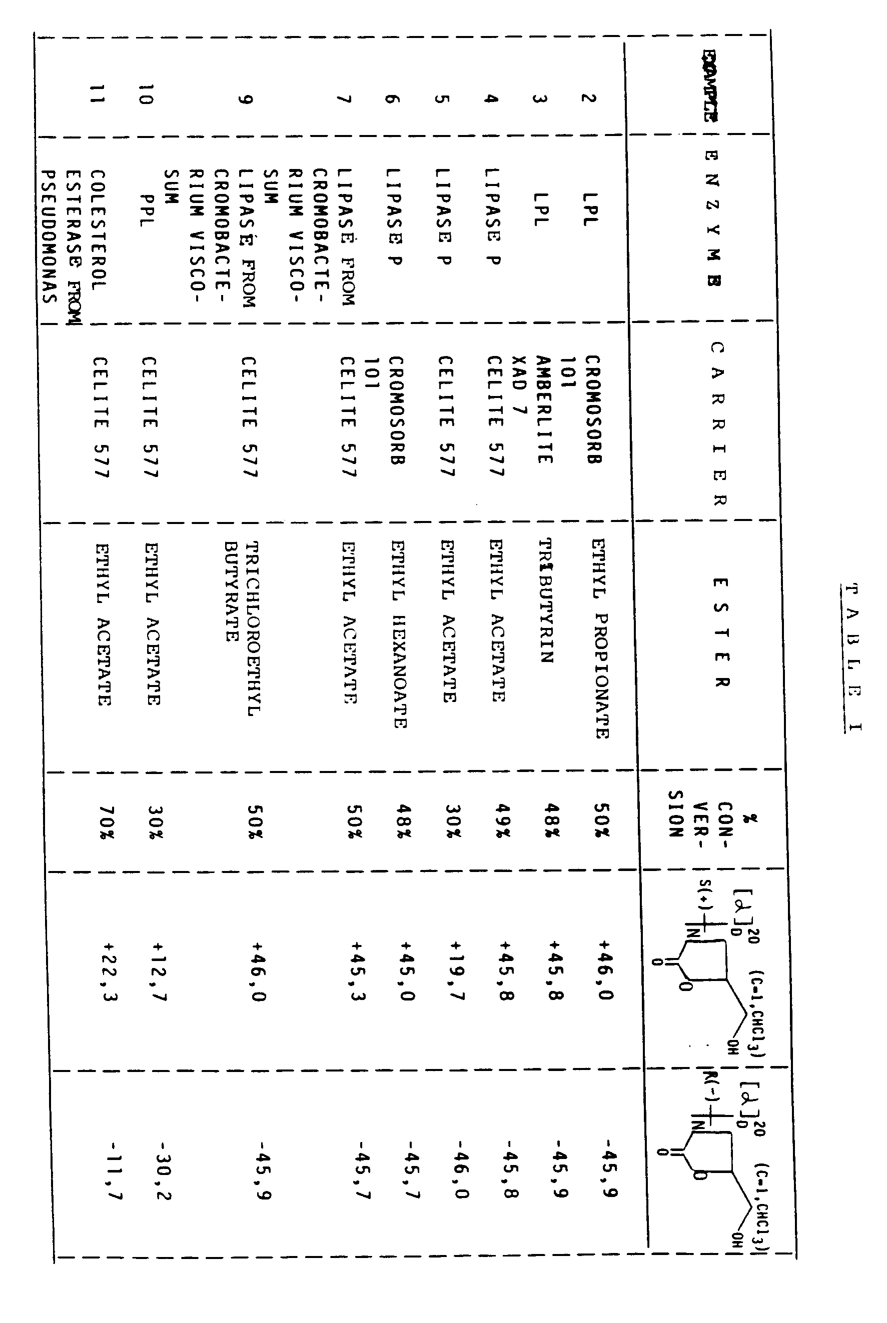

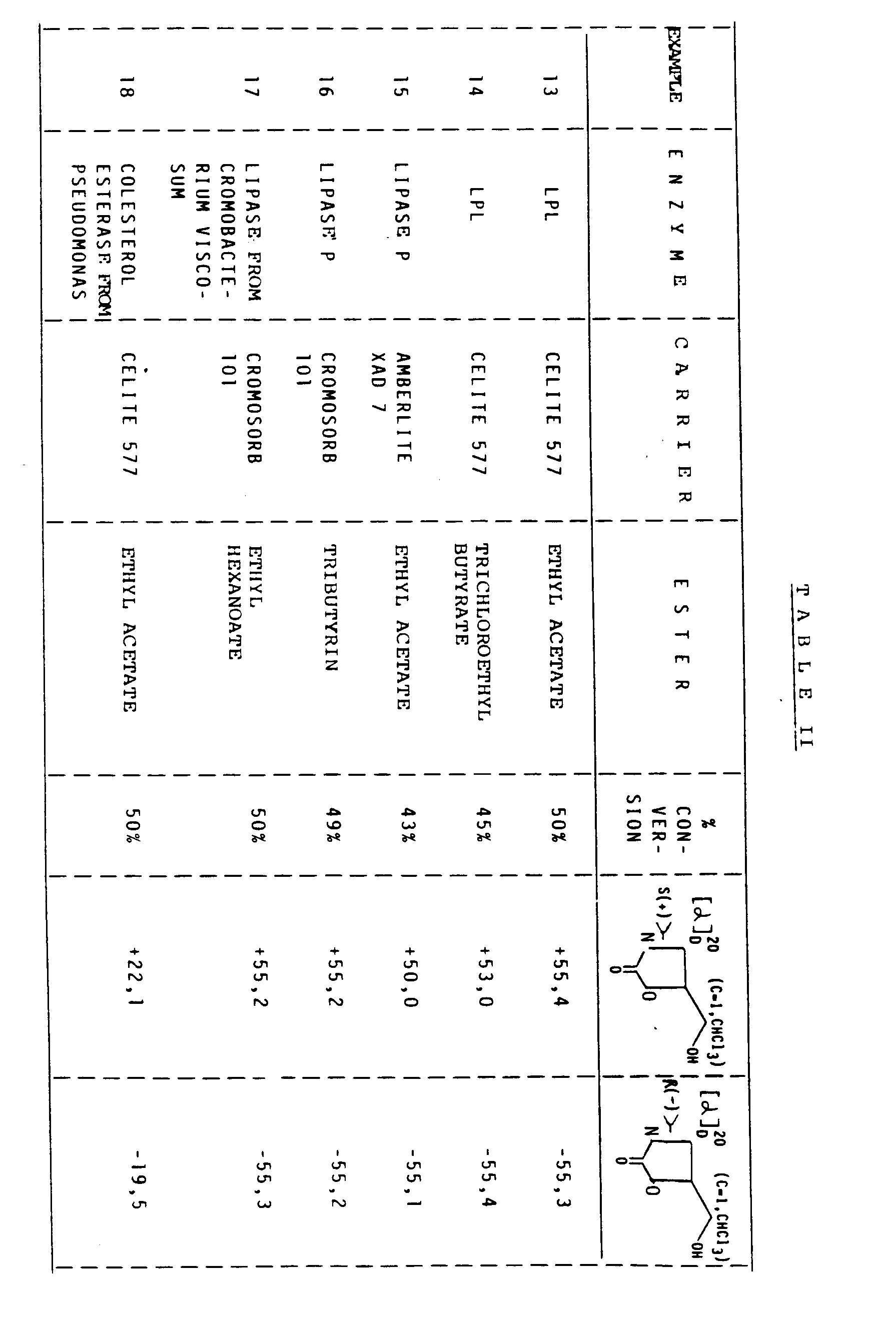

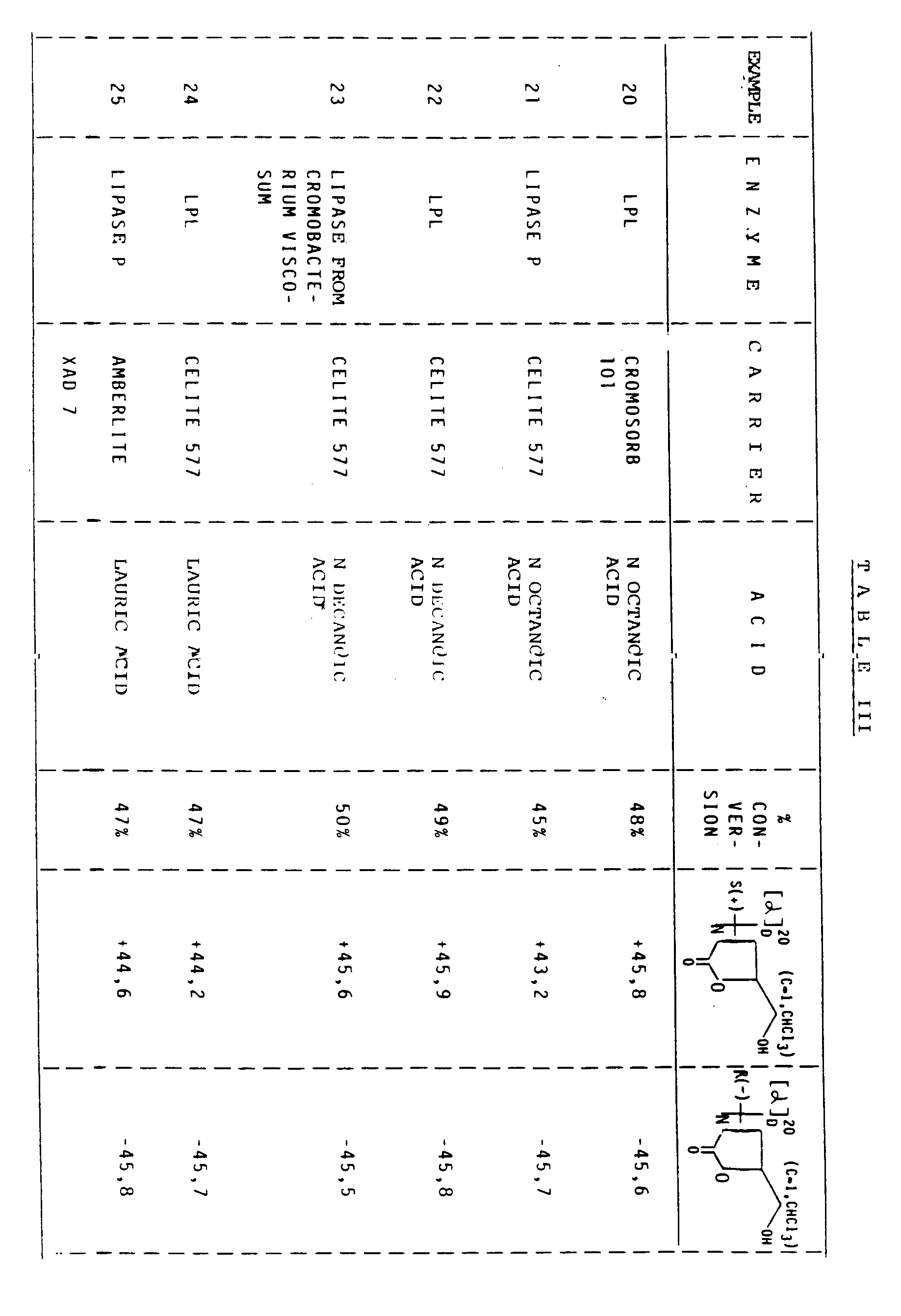

## Claims

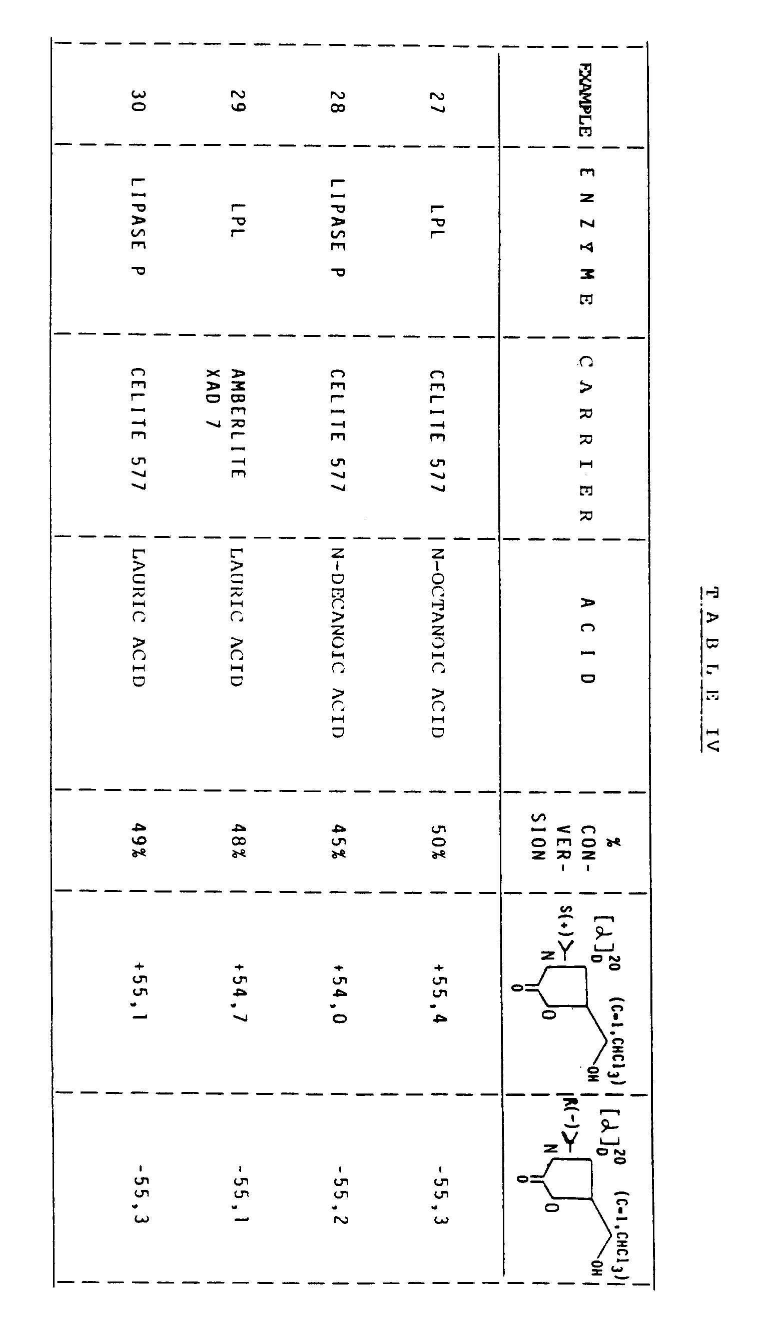

1. A process for the separation of a racemic mixture of the S(+) and R(-) optical isomers of an oxazolidinone compound of the formula: 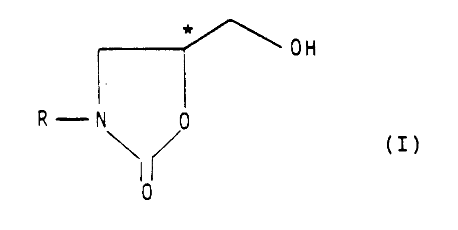 (in which R is a straight or branched C₁-C₈ alkyl group) which process comprises reacting the racemic 3-alkyl-5-hydroxy-methyl-oxazolidin-2-one derivative of formula (I) with an esterifying compound which is
(a) an ester of the formula: 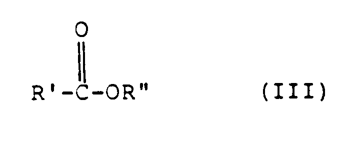 (in which Rʹ is a straight or branched C₁-C₁₀ alkyl or alkenyl group, and Rʺ is a straight or branched C₁-C₄ alkyl or alkenyl group, a haloalkyl group or a diacylglycerolic group),
(b) an acid of the formula:
Rʺʹ-COOH (IV)
(in which Rʺʹ is a straight or branched C₁-C₂₀ alkyl or alkenyl group) or
(c) an anhydride of the formula: 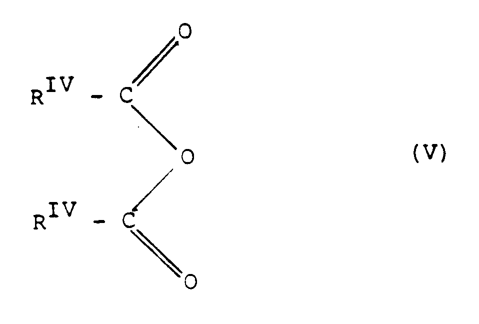 (in which R^{IV} is a straight or branched C₁-C₆ alkyl group)
the reaction being carried out in the presence of an enzyme, immobilized on a porous carrier, capable of selectively giving rise to the esterification of the R(-) isomer, while leaving the S(+) isomer of the racemic starting compound of formula (I) substantially unchanged;
and separating the S(+) isomer.

2. A process according to claim 1, characterized in that the reaction is carried out using an ester of formula (III) in a molar ratio of ester (III) to compound of formula (I) of from 10:1 to 500:1, preferably from 50:1 to 200:1.

3. A process according to claim 1 or claim 2 characterized in that the reaction is carried out using an ester of formula (III) or an acid of formula (IV) and the reaction is carried out at a temperature of from 0 to 50°C preferably from 20 to 30°C.

4. A process according to claim 1 characterized in that the reaction is carried out using a carboxylic acid of formula (IV) or an anhydride of formula (V) and the molar ratio of the carboxylic acid (IV) or the anhydride (V) to the compound of formula (I) is from 0.6:1 to 5:1, preferably from 0.8:1 to 1.5:1.

5. A process according to claim 1 or claim 4 characterized in that the reaction with the acid (IV) or the anhydride (V) is carried out in an aromatic hydrocarbon or halogenated aliphatic hydrocarbon solvent such as benzene, toluene, methylene chloride or chloroform.

6. A process according to any one of claims 1, 4 or 5, characterised in that there is used an anhydride of formula (V) and reaction is carried out at a temperature of from -10° to 30°C, preferably from 0 to 20°C.

7. A process according to any one of the preceding claims characterized in that the molar concentration of the compound of formula (I) in the reaction mixture is from 0.01 to 2 moles, preferably from 0.1 to 1 mole.

8. A process according to any one of the preceding claims characterized in that the enzyme is a lipase selected from LPL, LIPASE P, LIPASE from Chromobacterium viscosum and LIPASE PL 266.

9. A process according to any one of the preceding claims characterized in that the weight ratio of enzyme to compound of formula (I) is from 1:1 to 1:2000.

## Patentansprüche

1. Verfahren zur Trennung einer racemischen Mischung von optischen S(+)- und R(-)-Isomeren von einer Oxazolidinonverbindung der Formel: 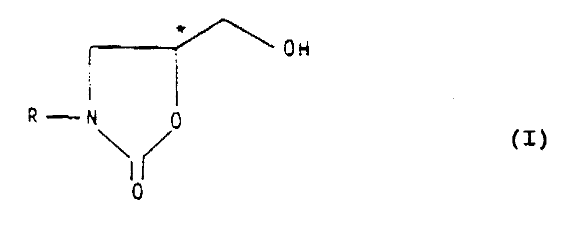 (worin R eine gerad- oder verzweigtkettige C₁-C₈-Alkylgruppe ist), wobei das Verfahren das Umsetzen des racemischen 3-Alkyl-5-hydroxy-methyl-oxazolidin-2-on-Derivates der Formel (I) mit einer veresternden Verbindung, die
(a) ein Ester der Formel: 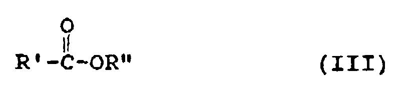 ist, (worin R' eine gerad- oder verzweigtkettige C₁-C₁₀-Alkyl-oder Alkenylgruppe und R" eine gerad- oder verzweigtkettige C₁-C₄-Alkyl- oder Alkenylgruppe, eine Halogenalkylgruppe oder eine Diacylglyceringruppe ist),
(b) eine Säure der Formel:
R'''-COOH (IV)
ist, (worin R''' eine gerad- oder verzweigtkettige C₁-C₂₀-Alkyl- oder Alkenylgruppe ist) oder
(c) ein Anhydrid der Formel: 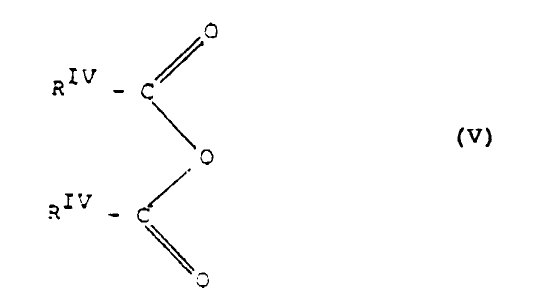 ist, (worin R^{IV} eine gerad- oder verzweigtkettige C₁-C₆-Alkylgruppe ist,
und die Umsetzung in Anwesenheit eines Enzyms, immobilisiert auf einem porösen Träger, erfolgt, das die Fähigkeit zur selektiven Veresterung des (R(-)-Isomers besitzt, während das S(+)-Isomer der racemischen Ausgangsverbindung der Formel (I) im wesentlichen unverändert bleibt;
und Abtrennung des S(+)-Isomers
umfaßt.

2. Verfahren nach Anspruch 1, dadurch gekennzeichnet, daß die Umsetzung unter Verwendung eines Esters der Formel (III) in einem Molverhältnis von Ester (III) zu Verbindung der Formel (I) von 10:1 bis 500:1, vorzugsweise von 50:1 bis 200:1, erfolgt.

3. Verfahren nach Anspruch 1 oder 2, dadurch gekennzeichnet, daß die Umsetzung unter Verwendung eines Esters der Formel (III) oder einer Säure der Formel (IV) bei einer Temperatur zwischen 0 bis 50° C, vorzugsweise zwischen 20 bis 30°C, erfolgt.

4. Verfahren nach Anspruch 1, dadurch gekennzeichnet, daß die Umsetzung unter Verwendung einer Carbonsäure der Formel (IV) oder eines Anhydrids der Formel (V) erfolgt, wobei das Molverhältnis der Carbonsäure (IV) oder des Anhydrids (V) zu der Verbindung der Formel (I) von 0,6:1 bis 5:1, vorzugsweise von 0,8:1 bis 1,5:1, beträgt.

5. Verfahren nach Anspruch 1 oder 4, dadurch gekennzeichnet, daß die Umsetzung mit der Säure (IV) oder dem Anhydrid (V) in einem aromatischen Kohlenwasserstoff- oder halogenisierten aliphatischen Kohlenwasserstofflösungsmittel wie Benzol, Toluol, Methylenchlorid oder Chloroform erfolgt.

6. Verfahren nach einem der Ansprüche 1, 4 oder 5, dadurch gekennzeichnet, daß ein Anhydrid der Formel (V) verwendet wird und die Umsetzung bei einer Temperatur von -10° bis 30°C, vorzugsweise von 0 bis 20° C erfolgt.

7. Verfahren nach einem der vorhergehenden Ansprüche, dadurch gekennzeichnet, daß die molare Konzentration der Verbindung der Formel (I) in der Reaktionsmischung von 0,01 bis 2 Mol, vorzugsweise von 0,1 bis 1 Mol, beträgt.

8. Verfahren nach einem der vorhergehenden Ansprüche, dadurch gekennzeichnet, daß das Enzym eine Dipase, ausgewählt aus LPL, LIPASE P, LIPASE von Chromobacterium viscosum und LIPASE PL 266 ist.

9. Verfahren nach einem der vorhergehenden Ansprüche, dadurch gekennzeichnet, daß das Gewichtsverhältnis von Enzym zu Verbindung der Formel (I) zwischen 1:1 bis 1:2000 beträgt.

## Revendications

1. Un procédé pour la séparation d'un mélange racémiques d'isomères optiques S(+) et R(-) d'un dérivé d'oxazolidinone de formule: 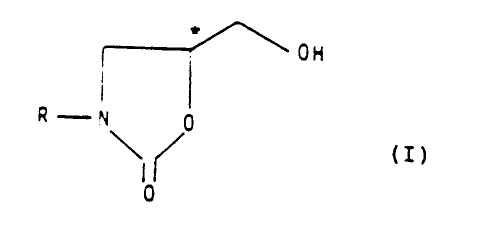 dans laquelle:
R est un radical alkyle en C₁ à C₈, linéaire ou ramifié, lequel procédé consiste à faire réagir le dérivé 3-alkyl-5-hydroxyméthyloxazolidin-2-one racémique de formule (I) avec un composé estérifiant qui peut consister en:
(a) un ester de formule: 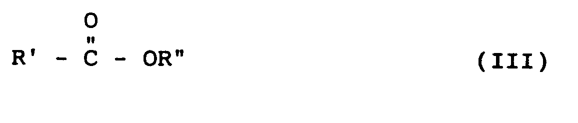 dans laquelle:
R' est un radical alkényle ou alkyle en C₁ à C₁₀, linéaire ou ramifié, et
R" est un radical alkényle ou alkyle en C₁ à C₄, linéaire ou ramifié, un radical haloalkyle ou un radical diacylglycérolique,
(b) un acide de formule:
R"' - COOH (IV)
dans laquelle:
R"' est un radical alkyle ou alkényle en C₁ à C₂₀, linéaire ou ramifié, ou
(c) un anhydride de formule: 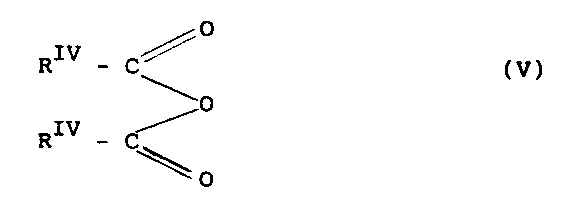 dans laquelle:
R^{IV} est un radical alkyle en C₁ à C₆, linéaire ou ramifié;
la réaction étant effectuée en présence d'une enzyme, immobilisée sur un support poreux, susceptible de donner lieu à l'estérification sélective de l'isomère R(-) tout en laissant l'isomère S(+) du composé racémique de départ de formule (I) substantiellement inchangé, ladite réaction étant suivie de la séparation de l'isomère S(+).

2. Un procédé selon la revendication 1, caractérisé en ce que la réaction fait emploi d'un ester de formule (III), les rapports molaires ester (III)/composé de formule (I) étant compris entre 10/1 et 100/1, de préférence 50/1 et 200/1.

3. Un composé selon la revendication 1 ou la revendication 2, caractérisé en ce que la réaction fait emploi d'un ester de formule (III) ou d'un acide de formule (IV) et que la réaction est effectuée à une température comprise entre 0 et 50°C, de préférence entre 20° et 30°C.

4. Un procédé selon la revendication 1, caractérisé en ce que la réaction fait emploi d'un acide carboxylique de formule (IV) ou d'un anhydride de formule (V) et en ce que le rapport molaire acide carboxylique (IV)/composé de formule (I) ou anhydride (V)/composé de formule (I) est compris entre 0,6/1 et 5/1, de préférence entre 0,8/1 et 5/1.

5. Un procédé selon la revendication 1 ou la revendication 4, caractérisé en ce que la réaction avec l'acide (IV) ou l'anhydride (V) est mise en oeuvre dans un solvant consistant en un hydrocarbure aromatique ou un hydrocarbure aliphatique halogéné tel que benzène, toluène, chlorure de méthylène ou chloroforme.

6. Un procédé selon l'une quelconque des revendications 1, 4 ou 5, caractérisé en ce que l'on utilise un anhydride de formule (V) et que la réaction est effectuée à une température comprise entre -10°C et 30°C, de préférence 0° et 20°C.

7. Un procédé selon l'une quelconque des revendications précédentes, caractérisé en ce que la concentration molaire du composé de formule (I) dans le mélange réactionnel est comprise entre 0,01 à 2 moles et de préférence comprise entre 0,1 à 1 mole.

8. Un procédé selon l'une quelconque des revendications précédentes, caractérisé en ce que l'enzyme est une lipase choisie dans le groupe comprenant LPL, LIPASE P, LIPASE obtenue à partir du Chromobacterium viscosum et LIPASE PL 266.

9. Un procédé selon l'une quelconque des revendications précédentes, caractérisé en ce que le rapport pondéral enzyme/composé de formule (I) est compris entre 1/1 et 1/2000.
